# EUROPEAN PATENT SPECIFICATION

(11) **EP 2 320 841 B1**
(45) Date of publication and mention of the grant of the patent: **16.10.2013**
(21) Application number: 09811815.1
(22) Date of filing: 28.08.2009
(51) Int. Cl.: A61F 7/00, A61F 7/02

(54) **FULL VIEW ACCESS UNDERBODY BLANKET**
UNTERBODEN DECKE MIT VOLLEM SICHTZUGANG
COUVERTURE DE DESSOUS DE TORSE À VISIBILITÉ TOTALE

(30) Priority: 04.09.2008 US 230777
(43) Date of publication of application: 18.05.2011
(73) Proprietor: Smiths Medical ASD, Inc., Rockland, MA 02370 (US)
(72) Inventor: ROBINSON, John, Imperial Beach CA 91932 (US); STEC, Alan, East Bridgewater MA 02333 (US)
(74) Representative: Hedges, Martin Nicholas
(86) International application number: PCT/US2009/004909
(87) International publication number: WO 2010/027440

(56) References cited:
- DE-U1- 20 202 811
- US-A- 4 867 230
- US-A1- 5 674 269
- US-A1- 5 773 275
- US-A1- 2007 244 532
- US-A1- 2007 244 532
- US-A1- 2008 288 034
- US-B1- 6 277 144

## Description

### Field of the Invention

The present invention relates to convective warming blankets and more particularly to an underbody blanket for warming the upper torso or upper body of a patient.

### Background of the Invention

There are currently upper body blankets that are used to warm the arms and upper body of a patient. One such blanket is disclosed in related U.S. patents 5,360,439, 5,384,924, 5,514,169 and 5,839,133. The blanket disclosed in the noted patents is placed over a patient, and has a recess portion for the head of the patient, so that the head of the patient is not covered by the blanket. Inlet ports are provided at the blanket at either side of the head of the patient. As far as can be ascertained from the disclosure of the noted patents, as the blanket is placed over the patient, heated air is mainly directed to the covered upper body of the patient, with the head of the patient exposed.

There is also disclosed an underbody blanket in U.S. patents 6,102,936 and 6,511,501. The blanket disclosed in those patents is a pediatric blankets that is to be placed underneath an infant patient, so that the entire body of the infant is exposed. There is a head portion whereupon the head of the patient lies. No heat is provided to the head of the patient, as all of the holes whereby heated air outputs from the blanket are located in the main body portion of the blanket.

US prior art document US 2007/244532, which is considered to represent the closest prior art, discloses An underbody blanket for supporting the upper body of a patient comprising an inflatable structure having a head portion for supporting the head of the patient, a body portion for supporting the upper torso of the patient, two arm portions, and sets of apertures provided along respective sections of the inflatable structure.

The blanket of the present invention is an underbody thermal convective blanket for the upper body or torso of the patient. The blanket is in the shape of a cross with a truncated vertical lower portion and a vertical upper portion onto which the head of the patient lies. Extending from the body portion of the blanket are two horizontal arm portions whereupon the arms of the patient rest. At each of the arm portions there is a transparent flap that may be moved to cover the arm of the patient. A T-shaped uninflated or non-inflated area is provided in the body and armed portions of the blanket whereupon the upper torso and arms of the patient may be positioned. A circular uninflated or non-inflated area in the head portion likewise provides a place onto which the head of the patient may be positioned.

To inflate the blanket, an inlet port is provided at an upper corner of the head portion of the blanket. Parallel rows of air holes or apertures are provided in the head portion for surrounding a major portion of the uninflated area at the head portion. Air apertures are also provided at the head portion, as well as the periphery of the inflated portion of the blanket that, except for the distal ends of the arm portions, surrounds the T-shaped uninflated area. Thus, air apertures are provided at the inflatable portion of the blanket both above and under the arms of the patient. Additional air apertures are provided at the junctions where the arm portions and the lower part of the body portion meet for outputting additional heated air to the patient. A number of sealing strips are formed at the head portion of the blanket for guiding the heated air input to the inlet port along the head portion as well as along and around the arm and body portions of the blanket.

To maintain warmth to the arms of the patient, a flexible flap, which may be transparent, clear or opaque, is attached to each of the arm portions. Optionally, fluid absorbent pads may be attachedly provided at the T-shaped uninflated area of the blanket for absorbing liquid that may be collected on the blanket. To enhance the flow of air along the blanket, all corners, both external and internal, of the blanket are rounded.

In accordance with the above, the present invention provides an under body blanket as defined in claim 1.

The present invention further provides a method of making an underbody convective blanket as defined in claim 9.

### Brief Description of the Figures

The present invention will become apparent and will best be understood by reference to the following description of the invention taken in conjunction with the accompanying drawings, wherein:

Fig. 1 is a plan view of the upper body underbody blanket of the present invention;

Fig. 2 is a cross-sectional view of the blanket along view 2-2 as shown in Fig. 1; and

Fig. 3 is another view of the blanket of the present invention having superposed thereon in phantom line the body of a patient.

### Detailed Description of the Invention

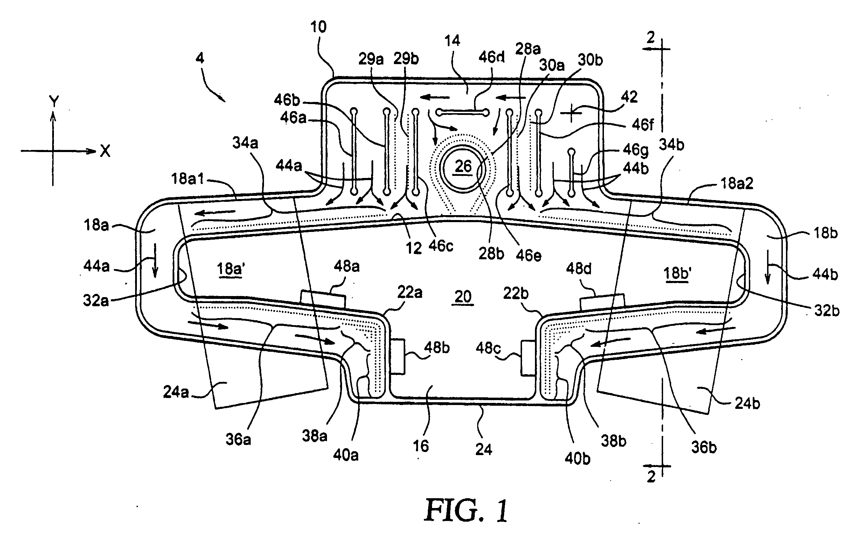

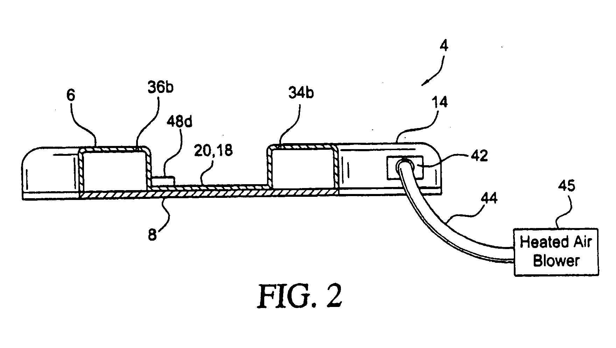

With reference to Figs. 1 and 2, the present invention convective warming blanket 4 is an inflatable structure made up of an upper air impermeable layer 6 which makes contact with a patient and a lower air impermeable layer 8. The upper and lower layers 6 and 8 are bonded together by a conventional sealing process at different areas of the blanket, as for example at the sealed outer periphery 10 and the sealed inner periphery 12. As shown, the overall blanket 4 is in the shape of a cross, with the lower vertical portion of the cross truncated.

As best shown in Fig. 1, blanket 4 includes a head portion 14, a body portion 16 and arm portions 18a and 18b, each of which extends from a corresponding side of body portion 16. The inflatable portion of the blanket may be identified by that area or portion sandwiched between the outer seal periphery 10 and the inner seal periphery 12. Thus, the blanket has an uninflated T-shaped area 20 at body portion 16 bounded by arm portions 18a and 18b, and a lower sealed edge 24. Arm portions 18a and 18b each are inflatable to be tube shaped. Area 20 includes the uninflated arm areas 18a' and 18b' each surrounded at three sides by the inflatable tubular arm portions 18a and 18b, respectively.

As further shown in Fig. 1, the uninflated arm areas 18a' and 18b' each are an extension of uninflated area 20 of the body portion 16 so that at the junction where the uninflated areas of the body and arm portions meet, two inner corners 22a and 22b are formed or defined by the inflatable portion of the blanket that further extends to the lowermost edge 24 of the blanket, which also may be referred to as the lowermost edge of area 20. These lower inflatable extensions of arm portions 18a and 18b may be referred to as the legs of the inflatable portion. At edge 24, the outer and inner sealed peripheries 10 and 12 are merged to form the lower boundary of the body portion for the blanket. To enhance the flow of air along the various portions of the blanket, all corners, both external and internal, of the blanket are rounded. See for example corners 22a and 22b.

Two flexible flaps 24a and 24b are connected to arm portions 18a and 18b at their respective upper edges 18a1 and 18a2. These flexible flaps may be made of transparent, clear or opaque PVC or other flexible materials and are movable between a position away from areas 18a1 and 18a2 to the positions as shown in Fig. 1 for covering the arms of a patient lying on blanket 4.

Blanket 4 further has a circular uninflated area 26 at its head portion 14 whereby the head of the patient is positionable. A double row of apertures 28a and 28b are provided at the head portion to surround the major portion of circular uninflated area 26. Additional rows of apertures 29a, 29b and 30a, 30b are provided longitudinally, along the y direction as shown in Fig. 1, at the head portion 14 of the blanket.

With respect to the body portion 16 and the arm portions 18a and 18b, except at the distal ends 32a and 32b of the arm portions 18a and 18b, there are a plurality of rows of air apertures provided along different sections on the inflatable upper layer 6 adjacent the inner periphery 12. As shown, respective rows of apertures 34a and 34b are provided at the inflatable portion adjacent to the inner periphery 12 that defines the uninflated T-shaped area 20. Note that the spacing of the apertures at rows 34a and 34b increases toward the center of the blanket but remains constant at the arm portions 18a and 18b.

Another set of respective rows of apertures 36a and 36b are provided to those sections of the inflatable portion adjacent to the inner periphery 12 that define the lower sections of inflatable arm portions 18a and 18b. The apertures are evenly spaced for the rows of apertures 36a and 26b. There are however a greater number of apertures provided at the cornered portions of the inflatable structure that define inner corners 22a and 22b of the uninflated area 20. These sets of apertures are designated 38a and 38b, respectively, in Fig. 1. A last dual set of apertures, each of which comprises a double row of apertures 40a and 40b, substantially extend from the inner corners 22a and 22b along the lower legs of the inflatable portion to the lower edge 24 of the blanket. Thus configured, air flow is promoted throughout the blanket, and a relatively constant temperature gradient is maintained for the air being circulated, even for the lower legs of the inflatable portion adjacent to body portion 16.

The temperature treated air, for example heated air, is input to the blanket 4 at inlet port 42, via for example an air hose 44 mated to the inlet port 42. The other end of air hose 44 in turn is connected to a heated air blower 45, for example an EQUATOR unit sold by the assignee of the present invention, so that heated air may be input to blanket 4 for inflating the blanket.

To enhance the circulation of air, and in particular directing the flow of air from inlet port 42 through head portion 14 and then via the paths as indicated by the directional arrows 44a and 44b to the remainder of the blanket, a number of sealing strips, for example 46a-46g, are provided at the head portion 14 of the blanket. The sealing strips 46a-46g are in spatial relationship to each other and are positioned such that the flow of air input from inlet port 42 may be directed, per paths 44a and 44b, to both arm portions 18a and 18b, and eventually from there to the lower legs of the inflatable portion that define the lower section of uninflated area 20 of body portion 16. With an increased number of apertures at inner corners 22a and 22b, per designated by aperture sections 38a and 38b, and the double rows of apertures 40a and 40b at the lower portion of body portion 16, more air is output at those portions of the blanket that are most remote from inlet port 42. As a result, the different portions of the upper torso or body of the patient lying on the blanket would be warmed substantially evenly, due to a greater amount of heated air being output from those portions of the blanket that are remote from the air inlet.

There are no apertures at distal ends 32a and 32b of the arm portions 18a and 18b. This is to ensure that those areas of the blanket onto which the hands and lower arms of the patient are to be positioned should not be heated, for it is highly likely that IV lines or other tubes and devices may need to be connected to those limbs of the patient. And these lines and devices, as well as the fluid in the IV lines, may be affected by heat. Thus, the hand areas of the blanket are designed not to have heated air output thereat.

Returning to the sealing strips, for directing the air flow in the blanket, strips 46g and 46f would direct the input air toward arm portion 18b. Moreover, with the sealing strip 46d configured in the x direction as shown, air flow is directed toward the area of head portion 14 away from air inlet port 42, so that the heated air may also be directed to the area of head portion 14 that contains strips 46a-46c, which may then further direct the heated air along paths 44a to arm portion 18a. Heated air also circulates about the uninflated area 26 at head portion 14, so that heated air is output from the rows of apertures 28a and 28b for warming the head of the patient.

Further shown with respect to Figs. 1 and 2 are optional fluid absorption pads 48a-48d that may be attached to different areas of the T-shaped uninflated area 20, for absorbing liquid that may be collected thereat, from either the patient or the medical personnel using it on the patient.

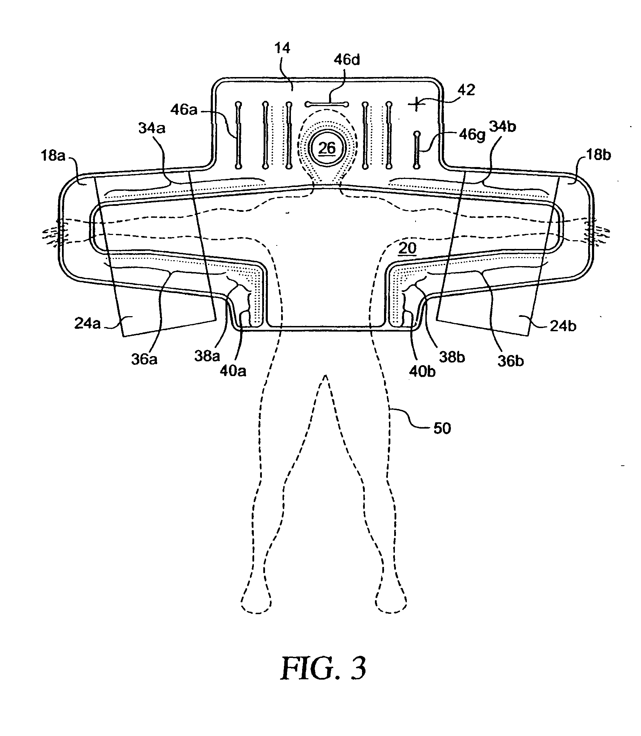

Fig. 3 shows the blanket of the present invention having positioned thereon an exemplar patient 50, shown in phantom line. As shown, the head of the patient rests on uninflated area 26 while his upper torso is positioned substantially on the uninflated area 20, with his arms extending and resting along arm areas 18a' and 18b'. The upper torso and arms of the patient accordingly are warmed by the heated air output from the respective sets of air outlet apertures 34a and 34b, 36a and 36b, 38a and 38b, and 40a and 40b provided at the inflatable upper layer 6 along the inner sealed periphery 12 that defines the uninflated area 20. Also, with the patient resting on the blanket, the head and upper torso of the patient are fully accessible to the medical personnel. To maintain the heat on the arms of the patient, flaps 24a and 24b are shown in Fig. 3 to have been moved to cover the arms of the patient.

## Claims

1. An underbody blanket (4) for supporting the upper body of a patient comprising an inflatable structure having a head portion (14) for supporting the head of the patient, a body portion (16) for supporting the upper torso of the patient, two arm portions, (18a, 18b) and sets of apertures (28a, 28b, 29a, 29b, 30a, 30b; 34a, 34b, 36a, 36b, 38a, 38b, 40a, 40b) provided along respective sections of the inflatable structure; the two arm portions having inflatable structures each extending from a corresponding side of said body portion for supporting the arms of the patient, and an inlet (42) provided at said head portion to enable temperature treated air to be input to said structure to flow along the respective sections of the inflatable structure **characterized in that** the underbody blanket indicates a substantially T shaped uninflatable area (20) in said body portion bounded by said head portion and extending along a portion of each of said arm portions whereon the upper torso and the arms of the patient are positionable, the inflatable structure defining two lower corners (22a, 22b) of the T shaped uninflatable area where said body and arm portions meet, a greater number of apertures (38a, 38b) provided at those sections of the inflatable structure that define said two lower corners (22a, 22b) than at other sections of the inflatable structure.

2. A blanket of claim 1, further comprising a substantially circular uninflatable area at said head portion where the head of the patient is positionable, a plurality of apertures surrounding at least a portion of said circular uninflatable area.

3. A blanket of claim 1, wherein the air input to the inlet is directed from said head portion to said body portion by flowing about the T shaped uninflatable area.

4. A blanket of claim 3 further comprising a plurality of sealing strips (46a-46g) at said head portion for directing the flow of input air to said arm and body portions of said blanket.

5. A blanket of claim 1, wherein the T shaped uninflatable area extends to the lowermost edge (24) of said body portion such that said arm portions of said blanket each comprise an inflatable tube surrounding an uninflatable area.

6. A blanket of claim 1, further comprising two flaps (24a, 24b) each at one of said arm portions (18a, 18b) of the blanket, each of said flaps movable to cover a corresponding arm of the patient lying on said blanket.

7. A blanket of claim 1, further comprising at least one liquid absorbent pad (48a, 48b) at the T shaped uninflatable area for absorbing liquid collected on the uninflatable area.

8. A blanket of claim 1, wherein all corners of said blanket are rounded.

9. A method of making an underbody convective blanket for warming the upper body of a patient lying thereon, comprising the steps of:
attaching air impermeable upper and lower layers (6,8) to form a scaled inflatable structure (4) having a head portion, a body portion and two arm portions each extending from a corresponding side of said body portion;
forming a substantially T shaped uninflatable area (20) bounded by at least said head portion and said arm portions whereupon the upper torso of the patient is positionable;
forming apertures (28a, 28b, 29a, 29b, 30a, 30b; 34a, 34b, 36a, 36b, 38a, 38b, 40a, 40b) in the layer of said structure that comes into contact with the torso of the patient along respective sections of the inflatable structure that define said body and arm portions, the inflatable structure defining two lower corners of the T shaped uninflatable area where said body and arm portions meet;
providing a greater number of apertures (38a, 38b) at those sections of the inflatable structure that define said two lower corners (22a, 22b) than at other sections of the inflatable structure; and
providing an inlet (42) at said head portion to enable temperature treated air input to said structure to flow from said head portion along the respective sections of the inflatable structure.

10. A method of claim 9, further comprising the step of forming respective sets of apertures at corresponding inflatable portions above and below the uninflatable areas of said arm portions.

11. A method of claim 9, further comprising the steps of:
forming a substantially circular uninflatable area (26) at the head portion where the head of the patient is positionable; and
providing a plurality of apertures (28a, 28b) about at least a portion of the circular uninflatable area to output heat to the head of the patient.

12. A method of claim 9 further comprising the steps of:
directing the air input to the inlet (42) to flow from said head portion to said body portion by routing the air flow about the T shaped uninflatable area.

13. A method of claim 9, further comprising the step of sealing said head portion with a plurality of strips (46a-46g)at spatial relationship to each other for directing the flow of input air to the inflatable arm and body portions of said blanket.

14. A method of claim 9, further comprising the step of attaching two flaps (24a, 24b) each at one of said arm portions (18a, 18b) of the blanket, each of said flaps movable to cover a corresponding arm of the patient lying on said blanket.

15. A method of claim 9, further comprising the step of rounding all corners of said blanket.

## Patentansprüche

1. Unterlegdecke (4) zum Tragen des Oberkörpers eines Patienten, umfassend ein aufblähbares Gebilde mit einem Kopfteil (14) zum Tragen des Kopfs des Patienten, einem Körperteil (16) zum Tragen des oberen Rumpfs des Patienten, zwei Armteilen (18a, 18b) und Sätzen von Öffnungen (28a, 28b, 29a, 29b, 30a, 30b; 34a, 34b, 36a, 36b, 38a, 38b, 40a, 40b), die entlang jeweiligen Abschnitten des aufblähbaren Gebildes bereitgestellt sind, wobei die zwei Armteile aufblähbare Gebilde, die sich jeweils von einer entsprechenden Seite des genannten Körperteils erstrecken, zum Tragen der Arme des Patienten haben, und einem an dem genannten Kopfteil bereitgestellten Einlass (42), um zu ermöglichen, dass temperaturbehandelte Luft in das genannte Gebilde eingegeben wird, um an den jeweiligen Abschnitten des aufblähbaren Gebildes entlang zu strömen, **dadurch gekennzeichnet, dass** die Unterlegdecke einen von dem genannten Kopfteil umgrenzten und an einem Teil von jedem der genannten Armteile entlang verlaufenden, im Wesentlichen T-förmigen nicht aufblähbaren Bereich (20) in dem genannten Körperteil aufzeigt, auf dem der obere Rumpf und die Arme des Patienten positioniert werden können, wobei das aufblähbare Gebilde zwei untere Ecken (22a, 22b) des T-förmigen nicht aufblähbaren Bereichs definiert, wo der/die genannte(n) Körper- und Armteil(e) aufeinandertreffen, wobei eine größere Anzahl von Öffnungen (38a, 38b) an jenen Abschnitten des aufblähbaren Gebildes, die die genannten zwei unteren Ecken (22a, 22b) definieren, bereitgestellt ist als an anderen Abschnitten des aufblähbaren Gebildes.

2. Decke nach Anspruch 1, die ferner einen im Wesentlichen kreisförmigen nicht aufblähbaren Bereich an dem genannten Kopfteil aufweist, wo der Kopf des Patienten positioniert werden kann, wobei eine Vielzahl von Öffnungen wenigstens einen Teil des genannten kreisförmigen nicht aufblähbaren Bereichs umgibt.

3. Decke nach Anspruch 1, wobei die in den Einlass eingegebene Luft durch Strömen um den T-förmigen nicht aufblähbaren Bereich von dem genannten Kopfteil zu dem genannten Körperteil gelenkt wird.

4. Decke nach Anspruch 3, die ferner eine Vielzahl von Dichtstreifen (46a - 46g) an dem genannten Kopfteil zum Lenken des Stroms eingegebener Luft zu den/dem genannten Armteilen und Körperteil der genannten Decke aufweist.

5. Decke nach Anspruch 1, wobei der T-förmige nicht aufblähbare Bereich bis an den untersten Rand (24) des genannten Körperteils verläuft, so dass die genannten Armteile der genannten Decke jeweils eine aufblähbare Röhre aufweisen, die einen nicht aufblähbaren Bereich umgibt.

6. Decke nach Anspruch 1, die ferner jeweils eine Lasche (24a, 24b) an jedem der genannten Armteile (18a, 18b) der Decke aufweist, wobei jede der genannten Laschen bewegt werden kann, um einen entsprechenden Arm des auf der genannten Decke liegenden Patienten zu bedecken.

7. Decke nach Anspruch 1, die ferner an dem T-förmigen nicht aufblähbaren Bereich wenigstens ein flüssigkeitsabsorbierendes Stück (48a, 48b) zum Absorbieren von auf dem nicht aufblähbaren Bereich angesammelter Flüssigkeit aufweist.

8. Decke nach Anspruch 1, wobei alle Ecken der genannten Decke abgerundet sind.

9. Verfahren zum Herstellen einer konvektiven Unterlegdecke zum Wärmen des Oberkörpers eines darauf liegenden Patienten, das die folgenden Schritte umfasst:
Anbringen luftundurchlässiger oberer und unterer Lagen (6, 8) zum Bilden eines abgedichteten aufblähbaren Gebildes (4) mit einem Kopfteil, einem Körperteil und zwei Armteilen, die sich jeweils von einer entsprechenden Seite des genannten Körperteils erstrecken,
Bilden eines wenigstens von dem genannten Kopfteil und den genannten Armteilen umgrenzten, im Wesentlichen T-förmigen nicht aufblähbaren Bereichs (20), auf dem der obere Rumpf des Patienten positioniert werden kann,
Bilden von Öffnungen (28a, 28b, 29a, 29b, 30a, 30b; 34a, 34b, 36a, 36b, 38a, 38b, 40a, 40b) in der Lage des genannten Gebildes, die mit dem Rumpf des Patienten in Berührung kommt, entlang jeweiligen Abschnitten des aufblähbaren Gebildes, die den/die genannten Körper- und Armteil(e) definieren, wobei das aufblähbare Gebilde zwei untere Ecken des T-förmigen nicht aufblähbaren Bereichs definiert, wo der/die genannte(n) Körper- und Armteil(e) aufeinandertreffen,
Bereitstellen einer größeren Anzahl von Öffnungen (38a, 38b) an jenen Abschnitten des aufblähbaren Gebildes, die die genannten zwei unteren Ecken (22a, 22b) definieren, als an anderen Abschnitten des aufblähbaren Gebildes und
Bereitstellen eines Einlasses (42) an dem genannten Kopfteil, um zu ermöglichen, dass in das genannte Gebilde eingegebene temperaturbehandelte Luft von dem genannten Kopfteil an den jeweiligen Abschnitten des aufblähbaren Gebildes entlang strömt.

10. Verfahren nach Anspruch 9, das ferner den Schritt des Bildens jeweiliger Sätze von Öffnungen an entsprechenden aufblähbaren Teilen ober- und unterhalb der nicht aufblähbaren Bereiche der genannten Armteile aufweist.

11. Verfahren nach Anspruch 9, das ferner die folgenden Schritte aufweist:
Bilden eines im Wesentlichen kreisförmigen nicht aufblähbaren Bereichs (26) an dem genannten Kopfteil, wo der Kopf des Patienten positioniert werden kann, und
Bereitstellen einer Vielzahl von Öffnungen (28a, 28b) um wenigstens einen Teil des kreisförmigen nicht aufblähbaren Bereichs zum Ausgeben von Wärme zu dem Kopf des Patienten.

12. Verfahren nach Anspruch 9, das ferner die folgenden Schritte aufweist:
Lenken der in den Einlass (42) eingegebenen Luft, so dass sie von dem genannten Kopfteil zu dem genannten Körperteil strömt, durch Leiten des Luftstroms um den T-förmigen nicht aufblähbaren Bereich.

13. Verfahren nach Anspruch 9, das ferner den Schritt des Abdichtens des genannten Kopfteils mit einer Vielzahl von Streifen (46a - 46g) in räumlicher Beziehung zueinander zum Lenken des Stroms eingegebener Luft zu den/dem aufblähbaren Arm- und Körperteil(en) der genannten Decke aufweist.

14. Verfahren nach Anspruch 9, das ferner den Schritt des Anbringens von jeweils einer Lasche (24a, 24b) an jedem der genannten Armteile (18a, 18b) der Decke aufweist, wobei jede der Laschen zum Bedecken eines entsprechenden Arms des auf der genannten Decke liegenden Patienten bewegt werden kann.

15. Verfahren nach Anspruch 9, das ferner den Schritt des Abrundens aller Ecken der genannten Decke aufweist.

## Revendications

1. Couverture de dessous de corps (4) pour soutenir le corps supérieur d'un patient, comprenant une structure gonflable avec une portion tête (14) pour soutenir la tête du patient, une portion corps (16) pour soutenir le torse supérieur du patient, deux portions bras (18a, 18b) et des ensembles d'ouvertures (28a, 28b, 29a, 29b, 30a, 30b ; 34a, 34b, 36a, 36b, 38a, 38b, 40a, 40b) prévus le long de sections respectives de la structure gonflable ; les deux portions bras possédant des structures gonflables, chacune s'étendant à partir d'un côté correspondant de ladite portion corps pour soutenir les bras du patient, et un orifice d'admission (42) prévu au niveau de ladite portion tête pour permettre à de l'air à température traitée d'être introduit dans ladite structure pour qu'il s'écoule le long des sections respectives de la structure gonflable, **caractérisée en ce que** la couverture de dessous de corps présente une zone non gonflable sensiblement en forme de T (20) dans ladite portion corps limitée par ladite portion tête et s'étendant le long d'une portion de chacune desdites portions bras sur laquelle le torse supérieur et les bras du patient peuvent être positionnés, la structure gonflable définissant deux coins inférieurs (22a, 22b) de la zone non gonflable en forme de T où se rencontrent lesdites portions corps et bras, un nombre d'ouvertures plus grand (38a, 38b) étant prévu au niveau de ces sections de la structure gonflable, lesquelles définissent lesdits deux coins inférieurs (22a, 22b), qu'au niveau d'autres sections de la structure gonflable.

2. Couverture selon la revendication 1, comprenant en outre une zone non gonflable sensiblement circulaire au niveau de ladite portion tête où la tête du patient peut être positionnée, alors qu'une pluralité d'ouvertures entoure au moins une portion de ladite zone non gonflable circulaire.

3. Couverture selon la revendication 1, l'air introduit dans l'orifice d'admission étant dirigé depuis ladite portion tête vers ladite portion corps en le faisant passer par écoulement autour de la zone non gonflable en forme de T.

4. Couverture selon la revendication 3, comprenant en outre une pluralité de bandes d'étanchéisation (46a-46g) au niveau de ladite portion tête afin de diriger le flux d'air introduit vers lesdites portions bras et corps de ladite couverture.

5. Couverture selon la revendication 1, la zone non gonflable en forme de T s'étendant vers le bord situé le plus bas (24) de ladite portion corps de sorte que lesdites portions bras de ladite couverture comportent chacune un tube gonflable lequel entoure une zone non gonflable.

6. Couverture selon la revendication 1, comprenant en outre deux rabats (24a, 24b) chacun au niveau d'une desdites portions bras (18a, 18b) de la couverture, chacun desdits rabats étant apte à être déplacé pour couvrir un bras correspondant du patient couché sur ladite couverture.

7. Couverture selon la revendication 1, comprenant en outre au moins un coussinet absorbeur de liquide (48a, 48b) au niveau de la zone non gonflable en forme de T afin d'absorber du liquide qui s'est accumulé sur la zone non gonflable.

8. Couverture selon la revendication 1, tous les coins de ladite couverture étant arrondis.

9. Procédé de fabrication d'une couverture convective de dessous de corps pour réchauffer le corps supérieur d'un patient qui est couché dessus, comprenant les étapes consistant à :
attacher des couches supérieure et inférieure imperméables à l'air (6, 8) pour constituer une structure gonflable étanche (4) avec une portion tête, une portion corps et deux portions bras, chacune s'étendant à partir d'un côté correspondant de ladite portion corps ;
former une zone non gonflable sensiblement en forme de T (20) limitée par au moins ladite portion tête et lesdites portions bras sur lesquelles le torse supérieur du patient peut être positionné ;
former des ouvertures (28a, 28b, 29a, 29b, 30a, 30b ; 34a, 34b, 36a, 36b, 38a, 38b, 40a, 40b) dans la couche de ladite structure qui vient au contact du torse du patient le long de sections respectives de la structure gonflable lesquelles définissent lesdites portions corps et bras, alors que la structure gonflable définit deux coins inférieurs de la zone non gonflable en forme de T où se rencontrent lesdites portions corps et bras ;
prévoir un nombre d'ouvertures plus grand (38a, 38b) au niveau de ces sections de la structure gonflable, lesquelles définissent lesdits deux coins inférieurs (22a, 22b), qu'au niveau d'autres sections de la structure gonflable ; et
prévoir un orifice d' admission (42), au niveau de ladite portion tête, pour permettre à de l'air à température traitée introduit dans ladite structure de passer par écoulement depuis ladite portion tête le long des sections respectives de la structure gonflable.

10. Procédé selon la revendication 9, comprenant en outre l'étape consistant à former des ensembles respectifs d'ouvertures au niveau de portions gonflables correspondantes, au-dessus et en dessous des zones non gonflables desdites portions bras.

11. Procédé selon la revendication 9, comprenant en outre les étapes consistant à :
former une zone non gonflable sensiblement circulaire (26) au niveau de la portion tête où la tête du patient peut être positionnée ; et
prévoir une pluralité d'ouvertures (28a, 28b) autour d'au moins une portion de la zone non gonflable circulaire afin de procurer de la chaleur à la tête du patient.

12. Procédé selon la revendication 9, comprenant en outre les étapes consistant à :
diriger l'air introduit dans l'orifice d'admission (42) pour le faire passer par écoulement depuis ladite portion tête vers ladite portion corps en acheminant le flux d'air autour de la zone non gonflable en forme de T.

13. Procédé selon la revendication 9, comprenant en outre l'étape consistant à étanchéifier ladite portion tête à l'aide d'une pluralité de bandes (46a-46g) suivant une relation spatiale l'une par rapport à l'autre, afin de diriger le flux d'air introduit vers les portions bras et corps gonflables de ladite couverture.

14. Procédé selon la revendication 9, comprenant en outre l'étape consistant à attacher deux rabats (24a, 24b) chacun au niveau d'une desdites portions bras (18a, 18b) de la couverture, chacun desdits rabats étant apte à être déplacé pour couvrir un bras correspondant du patient couché sur ladite couverture.

15. Procédé selon la revendication 9, comprenant en outre l'étape consistant à arrondir tous les coins de ladite couverture.
